Europäisches Patentamt

European Patent Office

Office européen des brevets

(11) Veröffentlichungsnummer: **0 389 753**
A2

## EUROPÄISCHE PATENTANMELDUNG

(21) Anmeldenummer: 90101770.7

(51) Int. Cl.5: **C07H 15/04**

(22) Anmeldetag: 30.01.90

(30) Priorität: 30.03.89 DE 3910269

(43) Veröffentlichungstag der Anmeldung:
03.10.90 Patentblatt 90/40

(84) Benannte Vertragsstaaten:
**DE FR GB IT**

(71) Anmelder: **HÜLS AKTIENGESELLSCHAFT**
**Patentabteilung / PB 15 - Postfach 13 20**
**D-4370 Marl 1(DE)**

(72) Erfinder: **Ripke, Norbert, Dr.**
**Hellweg 28**
**D-4358 Haltern(DE)**
Erfinder: **Hofmann, Peter, Dr.**
**Lavendelweg 28**
**D-4370 Marl(DE)**

(54) **Verfahren zur Herstellung von Alkylpolyglycosiden.**

(57)  2.1 Die Herstellung von Alkylpolyglycosiden von Sacchariden führt zu dunkel gefärbten Produkten. Hellfarbige Alkylpolyglycoside lassen sich daraus durch katalytische Hydrierung, durch Zusatz von Reduktionsmitteln oder durch Bleichung mit Wasserstoffperoxid gewinnen.

2.2 Durch Behandlung dunkel gefärbter Alkylpolyglycosidlösungen mit Ozon in einer Austauschersäule gelingt nun in einfacher Weise eine rückstandsfreie Bleichung und die Herstellung hellfarbiger Alkylpolyglycoside.

2.3 Herstellung hellfarbiger Alkylpolyglycoside für Haushalts- und Kosmetikartikel.

EP 0 389 753 A2

## Verfahren zur Herstellung von Alkylpolyglycosiden

Die Erfindung betrifft ein Verfahren, nach dem mit Hilfe von Bleichmitteln hellfarbige Alkylpolyglycoside hergestellt werden können.

Alkylpolyglycoside mit langkettigen Alkylresten können ganz oder teilweise aus nachwachsenden Rohstoffen hergestellt werden. Diese Alkylpolyglycoside gewinnen wegen ihrer interessanten Tensideigenschaften bei gleichzeitig sehr guter biologischer Abbaubarkeit zunehmend an Bedeutung. Für Anwendungen im Haushalt und im Kosmetikbereich müssen diese Produkte hohen ästhetischen Ansprüchen genügen. Man ist daher an Verfahren interessiert, nach denen Alkylpolyglycoside in transparenten, farblich schönen wäßrigen Lösungen hergestellt werden können.

Die Alkylpolyglycoside werden im allgemeinen aus Sacchariden und Alkoholen durch Glycosidierung und Umglycosidierung hergestellt. Die Produkte, die man dabei erhält, sind jedoch dunkel gefärbt.

Die Farbe kann nach US 4 762 918 durch katalytische Hydrierung verbessert werden.

Bei der Herstellung von langkettigen Alkylsacchariden können nach US 4 465 828 auch die Hydroxylpolycarbonsäuren Citronensäure, Weinsäure und Äpfelsäure zur Farbverbesserung beitragen.

Nach EP 0 077 167 können bei der Umsetzung von Alkoholen mit Aldosen oder Ketosen Reduktionsmittel, wie hypophosphorige Säure oder schwefelige Säure, zugesetzt werden. Dadurch wird die Farbe der Alkylglycoside verbessert.

Es sind auch vorbeugende Maßnahmen bekannt. So erhält man nach EP 0 102 558 farblich verbesserte $C_3$- bis $C_5$-Alkylglucoside, wenn man die Glucosidierung in Gegenwart eines Alkalisalzes einer Borsäure durchführt.

Nach EP 0 165 721 kann die Farbe der Produkte durch mehrstufige Bleichung mit Wasserstoffperoxid verbessert und durch Zusatz von Schwefeldioxid freisetzenden Verbindungen stabilisiert werden. Zu dem hier auch erwähnten Ozon werden keine weiteren Angaben gemacht.

Die katalytische Hydrierung ist wegen der hohen Katalysatorkosten und der schwierigen Reaktionsführung problematisch. Die Wasserstoffperoxid-Bleichung erfordert bei großtechnischen Verfahren die Lagerung und Handhabung großer Mengen an Peroxid. Bei allen festen und flüssigen Farbverbesserern ist es außerdem schwierig, diese Mittel nach beendeter Reaktion quantitativ zu entfernen.

Es bestand daher die Aufgabe, ein in Verfahrensweise und Handhabung vereinfachtes Verfahren zur Farbverbesserung von Alkylpolyglycosiden mit leicht entfernbaren Mitteln bereitzustellen.

Die Aufgabe wird erfindungsgemäß dadurch gelöst, daß man in einer Austauschersäule ein Gas, das über 5 % Ozon enthält, bei 20 bis 100 °C durch eine 20- bis 80%ige wäßrige Alkylpolyglycosidlösung mit einem pH-Wert von 3 bis 11 leitet.

Die hier eingesetzten Alkylpolyglycoside können durch Glycosidierung und Umglycosidierung hergestellt werden. Sie enthalten Alkylgruppen mit 8 bis 24 C-Atomen. Der mittlere Polymerisationsgrad liegt bei 1 bis 10. Die Glycosideinheiten können beispielsweise von Glucose, Mannose, Maltose oder Lactose hergeleitet werden.

Vorzugsweise werden die Alkylpolyglycoside als 40- bis 70%ige wäßrige Lösung eingesetzt, wobei der pH-Wert auf 4 bis 8 eingestellt wird. Die Bleichung findet vorzugsweise bei 60 bis 90 °C statt.

Als Bleichmittel können Ozon/Luft-Gemische eingesetzt werden, wobei man jedoch vorzugsweise ozonreiche Ozon/Sauerstoff-Gemische verwendet. In einer besonders bevorzugten Ausführungsform leitet man reines Ozon, das herstellungsbedingt bis zu 5 % Sauerstoff enthalten kann, durch die Alkylpolyglycosidlösung.

Die Bleichung erfolgt in einer Austauschersäule, die eine gute Durchmischung von Gas und Lösung ermöglichen soll. Geeignet sind beispielsweise Füllkörperkolonnen, Sieb- oder Glockenbodensäulen oder Blasensäulen, wobei die letztgenannten Säulen bevorzugt werden.

Vorzugsweise werden bei der Bleichung Gas und Lösung im Gegenstrom geführt. In einer besonders bevorzugten Ausführungsform wird die Ozonbleichung kontinuierlich durchgeführt. Dabei gibt man die Alkylpolyglycosidlösung oben auf die Säule, läßt die Lösung nach unten fließen und stellt bestimmte mittlere Verweilzeit ein. Gleichzeitig leitet man im unteren Bereich der Säule ozonhaltiges Gas ein, das dann der Lösung entgegensteigt.

Nach beendeter Bleichung kann man durch Destillation des Wassers direkt hellfarbige Alkylpolyglycoside erhalten. Als hellfarbig bezeichnen wir Produkte, deren 50%ige wäßrige Lösungen Farbzahlen von unter 40 mg $I_2$/100 ml aufweisen.

Durch einfaches Einleiten eines Gases in die zu bleichende Alkylpolyglycosidlösung kann nach dem erfindungsgemäßen Verfahren eine sehr gute Bleichwirkung erzielt werden. Das Bleichmittel verbleibt dabei auch bei Überdosierung nicht in der wäßrigen Lösung. Ozon ermöglicht eine rückstandfreie Bleichung. Während bei der Wasserstoffperoxidbleichung eine zu hohe Wasserstoffperoxiddosierung zu hohem Schaum und zu nicht tolerierbaren Restperoxidgehalten führt, wird ein derartiger Effekt bei einer Überdosierung von Ozon nicht beobachtet. Das erfindungsgemäße Verfahren ist deshalb einfach und wenig störanfällig.

In den folgenden Beispielen, die die Erfindung verdeutlichen sollen, werden die Farbzahlen mit Hilfe einer Vergleichsskala gemäß DIN 6 162 ermittelt.

Beispiel 1

In einen 1-1-Blasensäulenreaktor werden 650 g 50%ige wäßrige Lösung von Dodecylpolyglucosid (Polymerisationsgrad 1,8) mit einer Farbzahl von > 300 mg $I_2$/100 ml und einem pH-Wert von 7 vorgelegt. Durch diese Lösung wird bei 80 °C ein Ozon/Sauerstoff/Stickstoff-Gemisch mit ca. 8 Vol.-% Ozon so geleitet, daß pro Stunde 4 g Ozon in die Lösung eingetragen werden. Dabei sinkt der pH-Wert auf 4. Der pH-Wert wird dann mit Hilfe von Natronlauge bei 4 konstant gehalten.

Der Ozongehalt des Gasgemisches wird auf übliche Weise durch Titration mit Kaliumiodid und Natriumthiosulfat bestimmt.

Nach einem Verbrauch von 7,5 g Ozon wird die Reaktion nach ca. 110 Minuten durch Abstellen des Gasstromes beendet. Die vormals dunkelbraune Lösung ist nun klar und hellgelb gefärbt (Farbzahl von 7 bis 10 ml $I_2$/100 ml).

Beispiel 2

In einen 1,5-l-Blasensäulenreaktor wird eine 50%ige wäßrige Lösung von Dodecylpolyglucosid (Polymerisationsgrad 2,3) mit einer Farbzahl von > 300 mg $I_2$/100 ml und einem pH-Wert von 6,5 mit einer Strömungsgeschwindigkeit von 1 kg/h eingeleitet. Die mittlere Verweilzeit im Reaktor beträgt 1 Stunde. Bei 70 °C werden 12 g/h reines Ozon im Gegenstrom durch die Lösung geleitet. Der pH-Wert wird bei 4 konstant gehalten.

Die abfließende Lösung weist eine Farbzahl von 7 bis 10 mg $I_2$/100 ml auf.

Beispiel 3

Durch einen 1,5-l-Blasensäulenreaktor wird eine 70%ige wäßrige Lösung von Dodecylpolyglucosid (Polymerisationsgrad 1,3) mit einer Farbzahl von > 300 mg $I_2$/100 ml gepumpt. Die mittlere Verweilzeit beträgt 1 Stunde. Bei 80 °C werden 17 g/h reines Ozon im Gegenstrom durch die Lösung geleitet. Der pH-Wert wird bei 4 konstant gehalten.

Die den Reaktor verlassende Lösung hat eine Farbzahl von 20 bis 30 mg $I_2$/100 ml.

**Ansprüche**

1. Verfahren zur Herstellung hellfarbiger Alkylpolyglycoside mit Hilfe von Bleichmitteln,
dadurch gekennzeichnet,
daß man in einer Austauschersäule ein Gas, das über 5 % Ozon enthält, bei 20 bis 100 °C durch eine 20- bis 80%ige wäßrige Alkylpolyglycosidlösung mit einem pH-Wert von 3 bis 11 leitet.

2. Verfahren nach Anspruch 1,
dadurch gekennzeichnet,
daß man ein Ozon/Sauerstoffgemisch einleitet.

3. Verfahren nach Anspruch 1,
dadurch gekennzeichnet,
daß man mit reinem Ozon bleicht.

4. Verfahren nach Anspruch 1,
dadurch gekennzeichnet,
daß man die Bleichung bei 60 bis 90 °C und einem pH-Wert von 4 bis 8 durchführt.

5. Verfahren nach Anspruch 1,
dadurch gekennzeichnet,
daß man die Bleichung in einer Blasensäule durchführt.

6. Verfahren nach Anspruch 1,
dadurch gekennzeichnet,
daß man die Alkylpolyglycosidlösung und das ozonhaltige Gas im Gegenstrom führt.

7. Verfahren nach Anspruch 1,
dadurch gekennzeichnet,
daß man die Bleichung kontinuierlich durchführt.